# EUROPEAN PATENT SPECIFICATION

(11) **EP 3 972 861 B1**
(45) Date of publication and mention of the grant of the patent: **03.05.2023**
(21) Application number: 21727937.1
(22) Date of filing: 17.05.2021
(51) Int. Cl.: B60H 3/00, B61D 27/00

(54) **MICROBIOLOGICAL SANITIZATION AND AIR PURIFICATION SYSTEM FOR HVAC SYSTEM OF A RAILWAY VEHICLE**
MIKROBIOLOGISCHES HYGIENISIERUNGS- UND LUFTREINIGUNGSSYSTEM FÜR HLK-SYSTEM EINES SCHIENENFAHRZEUGES
SYSTÈME DE PURIFICATION D'AIR ET DE DÉSINFECTION MICROBIOLOGIQUE POUR SYSTÈME DE CVC DE VÉHICULE FERROVIAIRE

(30) Priority: 19.05.2020 IT 202000011512
(43) Date of publication of application: 30.03.2022
(73) Proprietor: STE-Sanitizing Technologies and Equipments S.r.l., 61020 Petriano (PU) (IT)
(72) Inventor: TORRESAN, Giuseppe, 11027 Saint-Vincent (Aosta) (IT); ALIANO, Mattia Paolo, 61010 Tavullia (Pesaro- Urbino) (IT)
(74) Representative: Verdecchia, Valerio
(86) International application number: PCT/IB2021/054194
(87) International publication number: WO 2021/234529

(56) References cited:
- EP-A1- 2 999 066
- WO-A1-2011/061913
- CN-A- 104 986 014
- CN-A- 107 351 651
- CN-U- 202 086 832
- JP-A- 2000 071 758
- KR-B1- 101 588 833

## Description

This invention relates to a purification and microbiological sanitization system for application to an HVAC system of a railway vehicle, for the removal or reduction of polluting volatile components and pathogens.

In general, within the scope of this description and the appended claims, a pathogen is understood to mean any biological or microbiological agent, such as a bacterium, a virus, a parasite, a prion, a fungus or the like or equivalent, whether proteinaceous or single-cell or multicellular.

"Purification and microbiological sanitization" of the air flow in this description and in the appended claims refers to the operation of subjecting a volume of air to a treatment process in order to damage, kill, sterilize, inactivate or otherwise render harmless a significant majority of the pathogens possibly contained therein, as well as removing as much as possible as many volatile or polluting components contained, suspended or transported by the air, substantially by carrying out decontamination operations, or so-called partial sterilization, or disinfection, microbiological sanitization, and purification.

"HVAC system" refers to a heating, ventilation, or air conditioning system or a system adapted to perform at least one of the functions listed above.

In the following description, for simplicity, reference will be made to the use in reference to a coach of a railway vehicle, but, obviously, the invention may be similarly implemented in reference to a coach, or to a part thereof, or to a cabin, of a tram vehicle, metropolitan railway or similar vehicle.

The growing increase in drug resistance due to the abuse of antibiotics (which causes about 700,000 deaths a year worldwide), the emergence of microorganisms with high mortality rate (e.g. tuberculosis, meningitis, etc.), the onset of new pathogens with high pandemic risk (MERS, SARS, Covid-19), in addition to the decrease in disinfections of vehicles of means of transport in order to contain operating costs, the slowness of the standard sanitation procedures that are implemented only for train shutdowns for second level maintenance (RO-RG), the overcrowding of coaches at peak times and finally the current presence of filtering systems with a permissive cut-off (Hepa filters > 0.5 µm), are all risk factors that draw the attention of the inventor to the need to provide means of transport, particularly in the railway sector, with disinfection or microbiological sanitization and air purification systems that may operate continuously with high efficiency. A known air purification and micro-biological sanitization system for a HVAC system of a railway vehicle is known, for example, from KR 101 588 833 B1.

The object of this invention is to provide such a purification and microbiological sanitization system, and in particular an improved purification and microbiological sanitization system with respect to the prior art.

This and other objects are fully achieved according to this invention by a purification and microbiological sanitization system as defined in the appended independent claim 1.

Advantageous embodiments of the purification and microbiological sanitization system according to the invention are specified in the dependent claims, the content of which is to be understood as an integral part of the description that follows.

In summary, the invention is based on the idea of providing a purification and microbiological sanitization system for a heating, ventilation, or air conditioning system of a railway vehicle or the like, the purification and microbiological sanitization system comprising:
a casing adapted to define therein a purification and microbiological sanitization chamber and having an inlet opening adapted to allow the inflow of air into the purification and microbiological sanitization chamber and an outlet opening adapted to allow the outflow of air from the purification and microbiological sanitization chamber;
an ionizer device adapted to at least partially ionize the air contained within said purification and microbiological sanitization chamber of the casing; an electronic control unit configured to control the purification and microbiological sanitization system; and
an air quality sensor adapted to measure a concentration of particulate matter and a concentration of carbon dioxide in the air at said outlet opening, to generate a respective measurement signal representative of this measurement, and to transmit it to said electronic control unit,

characterized in that it further comprises
   a plurality of light-emitting diodes, each adapted to generate a germicidal ultraviolet radiation having a wavelength between 100 and 300 nanometers and to emit said radiation inside said chamber for purification and microbiological sanitization of the casing;
wherein the electronic control unit is configured to control the radiant power of said at least one light-emitting diode as a function of the measurement signal transmitted by the air quality sensor,
wherein said microbiological sanitation and purification chamber is divided into a plurality of ducts arranged side by side, and
wherein at least one respective light-emitting diode of said plurality of light-emitting diodes is arranged inside each of said ducts.

By virtue of such a configuration of the purification and microbiological sanitization system, it is possible to ensure adequate purification and microbiological sanitization, or decontamination and purification, of the air inside a coach, including therein the driver's cabin, or the engine driver's cabin, of a railway vehicle on a continuous basis.

Preferably, the purification and microbiological sanitization system further comprises environmental sensors (humidity, temperature, possibly other volatile organic components) adapted to measure respective parameters and to transmit the result of this measurement to the electronic control unit to optimize control.

In this way, it is possible to continuously monitor the outcome of the purification and microbiological sanitization operation carried out by the purification and microbiological sanitization system and to adjust the control parameters (for example, the radiant power emitted by at least one light-emitting diode with germicidal ultraviolet emission, or the power supply to the ionizer device) in order to achieve the desired balance between air quality, level of purification and microbiological sanitization or disinfection and energy purification and microbiological sanitization chamber of the casing; and
an electronic control unit configured to control the purification and microbiological sanitization system;
   characterized in that it further comprises
an air quality sensor adapted to measure a concentration of particulate matter and a concentration of carbon dioxide in the air at said outlet opening, to generate a respective measurement signal representative of this measurement, and to transmit it to said electronic control unit,
   wherein the electronic control unit is configured to control the radiant power of said at least one light-emitting diode as a function of the measurement signal transmitted by the air quality sensor,
   wherein said microbiological sanitation and purification chamber is divided into a plurality of ducts arranged side by side, and
   wherein at least one respective light-emitting diode of said plurality of light-emitting diodes is arranged inside each of said ducts.

By virtue of such a configuration of the purification and microbiological sanitization system, it is possible to ensure adequate purification and microbiological sanitization, or decontamination and purification, of the air inside a coach, including therein the driver's cabin, or the engine driver's cabin, of a railway vehicle on a continuous basis.

Preferably, the purification and microbiological sanitization system further comprises environmental sensors (humidity, temperature, possibly other volatile organic components) adapted to measure respective parameters and to transmit the result of this measurement to the electronic control unit to optimize control.

In this way, it is possible to continuously monitor the outcome of the purification and microbiological sanitization operation carried out by the purification and microbiological sanitization system and to adjust the control parameters (for example, the radiant power emitted by at least one light-emitting diode with germicidal ultraviolet emission, or the power supply to the ionizer device) in order to achieve the desired balance between air quality, level of purification and microbiological sanitization or disinfection and energy consumption of the purification and microbiological sanitization system.

Further features and advantages of this invention will be clarified by the detailed description that follows, given purely by way of non-limiting example in reference to the accompanying drawings, wherein:
Fig. 1 is a partial perspective view in cross section of the purification and microbiological sanitization system according to an embodiment of the invention, wherein the ducts have been omitted for clarity;
Fig. 2 is a schematic side view of a railway vehicle to which the invention may be applied;
Fig. 3 is a block diagram of the purification and microbiological sanitization system of Fig. 1, wherein the blank arrows indicate the direction of the flow of air that is sanitized and purified; and
Fig. 4 is a perspective view of the purification and microbiological sanitization system of Fig. 1, wherein the ducts are also visible.

In reference to the figures, the purification and microbiological sanitization system according to the invention is generally indicated with 10. The purification and microbiological sanitization system 10 is adapted to be applied to a heating, ventilation, or air conditioning system 12 of a railway vehicle V, or to a single coach C of said railway vehicle V.

The purification and microbiological sanitization system 10 according to the invention essentially comprises a casing 14, a plurality of light-emitting diodes LED, an ionizer device 16, an electronic control unit ECU, and an air quality sensor 18.

The casing 14 is made as a hollow body, preferably with a tubular structure or a duct, substantially in the form of a frame, or a housing.

Preferably, the casing 14 is made of a non-magnetic metallic material, even more preferably of aluminum.

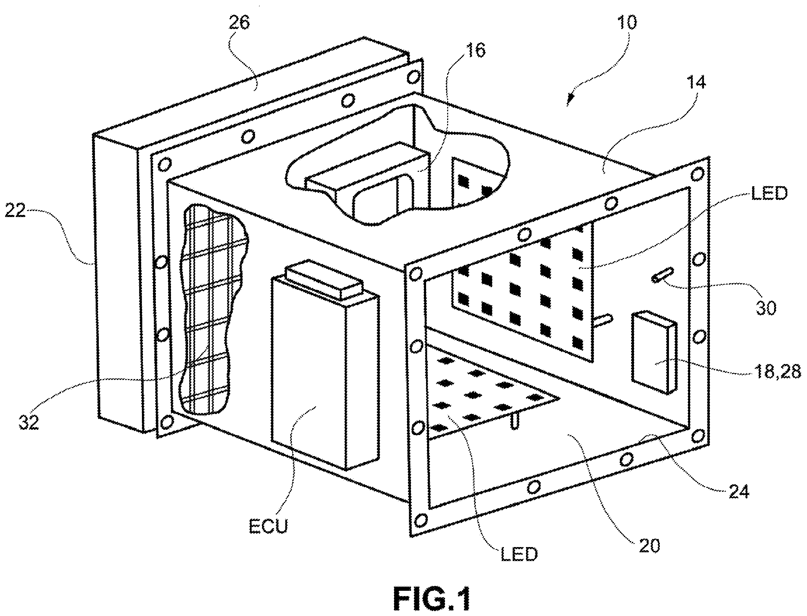

In the embodiment shown in Fig. 1, the casing 14 is made in the form of a rectilinear duct with a quadrangular cross section, but it is not excluded that it may take on different shapes, with a lattice of parallel inner ducts, or in particular that it may comprise a duct of the heating, ventilation, or air conditioning system 12 of the railway vehicle V.

The casing 14 is adapted to define therein a purification and microbiological sanitization chamber 20 and has an inlet opening 22 adapted to allow the inflow of air into said purification and microbiological sanitization chamber 20 to be sanitized and purified, i.e., decontaminated, and an outlet opening 24 adapted to allow the outflow of sanitized and purified, i.e., decontaminated, air from said purification and microbiological sanitization chamber 20.

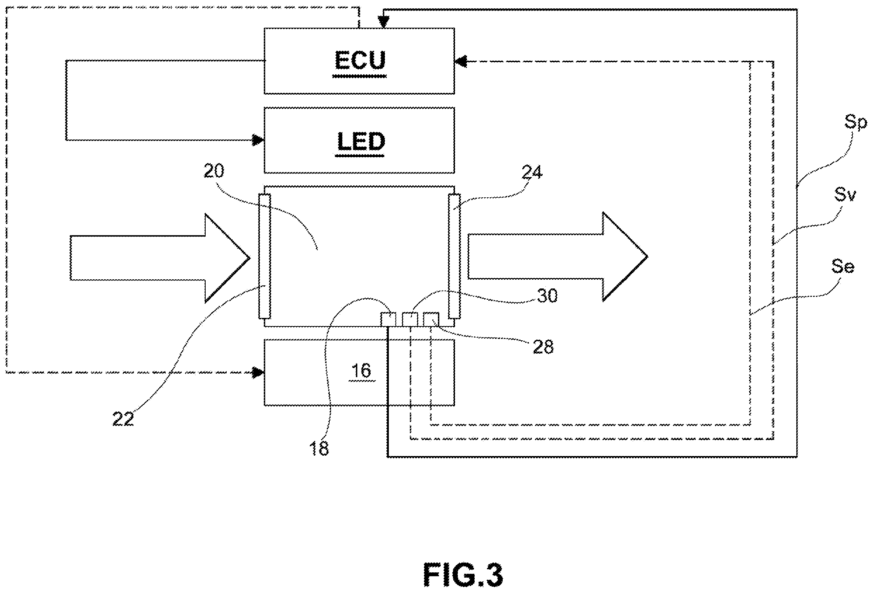

An air flow to be sanitized and purified, i.e., decontaminated, is made to flow inside the casing 14, in a manner known per se, according to the flow direction indicated by the blank arrows in Fig. 3.

Advantageously, the purification and microbiological sanitization chamber 20 is internally coated at least partially with a material reflecting UV-C ultraviolet radiation, such as, for example, Teflon.

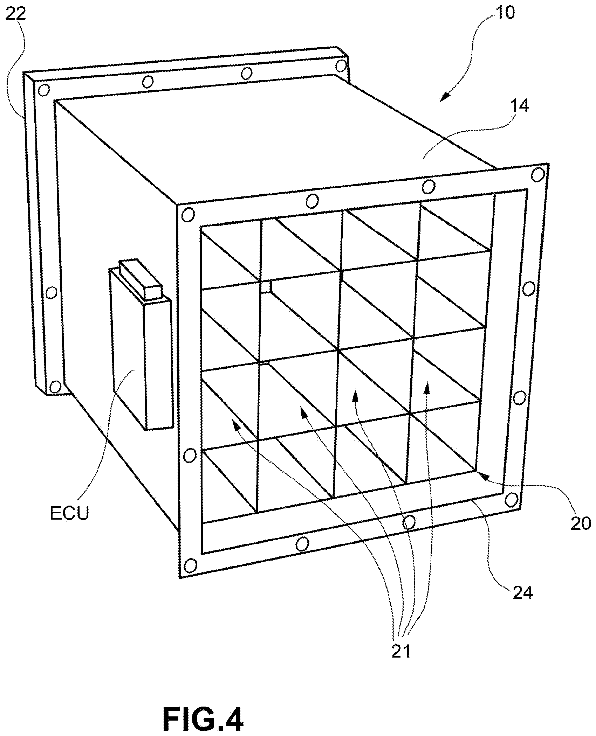

The purification and microbiological sanitization chamber 20 is internally divided into a plurality of ducts 21, as shown in Fig. 4. The ducts 21 are arranged side by side and are preferably also made with a quadrangular cross section.

Preferably, the internal surface of each of these ducts 21 is coated with a material reflecting UV-C ultraviolet radiation, such as, for example, aluminum and/or polytetrafluoroethylene.

Advantageously, at least one filter 26 may be arranged at the inlet opening 22 so as to carry out a first mechanical filtering of the air entering the purification and microbiological sanitization chamber 20. Preferably, this filter 26 may be classified as category G4 according to regulatory standard EN 779:2012.

The light-emitting diodes LED are adapted to generate a germicidal ultraviolet radiation and to emit it within the purification and microbiological sanitization chamber 20 of the casing 14. This germicidal ultraviolet radiation has a wavelength between 100 and 300 nanometers, preferably between about 255 nanometers and about 300 nanometers. In a manner known per se, the ultraviolet radiation of the UV-C type has a particular germicidal efficacy, that is, it is suitable for killing or inactivating any microbiological pathogens present in the air, since these wavelengths have the greatest absorption by nucleic acids: the absorption by nucleic acids of this radiation may cause genetic defects, including the formation of pyrimidine dimers, which may prevent the replication of the nucleic acid and the expression of the proteins necessary for the life cycle of the microorganism, resulting in direct death or inactivation of the microorganism.

As may be seen in Fig 1, the purification and microbiological sanitization system 10 may comprise a certain number of light-emitting diodes LED; in the example shown, there are four panels each comprising a plurality of light-emitting diodes LED (up to 144 light-emitting diodes LED in total) emitting germicidal ultraviolet radiation, the four panels being respectively arranged on four internal walls of the casing 14, inside the purification and microbiological sanitization chamber 20. In a manner known per se, the presence of a plurality of light-emitting diodes LED allows the appropriate germicidal efficacy to be maintained even in the event of failure of one thereof, ensuring the necessary redundancy.

The arrangement of the same light-emitting diodes LED, possibly grouped into modules, is obtained in such a way as to maximize the reflective effect of the germicidal ultraviolet radiation UV-C emitted thereby and thus to maximize the radiant power that reaches the inner volume of the purification and microbiological sanitization chamber 20.

In any case, the arrangement of light-emitting diodes LED is such as to ensure that the emitted radiation reaches for the most part the purification and microbiological sanitization chamber 20 and is not dispersed inside the coach C of the railway vehicle V, where it could be a source of danger for the crew or passengers.

In particular, at least one respective light-emitting diode LED of said plurality of light-emitting diodes LED is arranged inside each duct 21. Preferably, more than one light-emitting diode LED is disposed within each duct 21. In an embodiment, in each duct 21, at least one light-emitting diode LED is arranged at a mid-section of the respective duct 21, that is, it is arranged at approximately half the length of the duct 21.

Advantageously, the light-emitting diodes LED of said plurality of light-emitting diodes LED are connected in series with each other.

The ionizer device 16 is adapted to at least partially ionize the air contained inside the purification and microbiological sanitization chamber 20 of the casing 14 and is arranged accordingly. The ionizer device 16 produces negative ions, which aid in the removal of gases, aerosols, allergens, and polluting particles. In effect, these negative ions bind to the particles present in the air and dispersed therein, such as dust, particulate matter, pathogens (or fragments thereof following exposure to germicidal ultraviolet radiation), and pollen, causing them to deposit on inner surfaces of the casing 14. In particular, the ionizer device 16 is adapted to ionize, through at least partial reduction, the ionizable compounds (dust, pollen, etc.) present in the air contained within said purification and microbiological sanitization chamber 20 of the casing 14.

In a preferable embodiment, the ionizer device 16 is adapted to ensure the generation of about 2000-4000 ions/cm³, so as to obtain an optimal balance between air purification and energy demand. The ionizer device 16 further generates a small fraction of ozone (about 0.1 ppm).

As may be seen in Fig. 1, the ionizer device 16 is arranged within the purification and microbiological sanitization chamber 20, but nothing prevents its arrangement from being different, as long as it is possible to ensure partial but sufficient ionization of the air contained in the purification and microbiological sanitization chamber 20.

In any case, the arrangement of the ionizer device 16 is such as to ensure that the ionized air is for the most part contained in the purification and microbiological sanitization chamber 20 and not present inside the coach C of the railway vehicle V, where it could be a source of danger for the crew or passengers.

Lastly, the purification and microbiological sanitization system 10 may also comprise a greater number of ionizer devices 16, without thereby departing from the scope of the invention.

In an embodiment which is particularly preferable and which has unexpectedly shown particularly high efficacy in achieving excellent levels of air quality, the ionizer device 16, the filter 26, and the plurality of ducts 21 are arranged in such a way that, during operation, the air introduced into the purification and microbiological sanitization chamber 20 passes in sequence through the ionizer device 16, the filter 26, and then the plurality of ducts 21. In this embodiment, even more advantageously, the filter 26 is made as a plate element, i.e., it comprises a partially permeable wall through which the air flow is made to pass, and which carries out the filtering and is irradiated by at least one respective light-emitting diode LED of said plurality of light-emitting diodes LED on each surface of said wall.

All components of the purification and microbiological sanitization system 10 that require electrical power to operate, including the light-emitting diodes LED, the ionizer device 16, and the electronic control unit ECU, are preferably powered by connection to the electrical power supplied to said railway vehicle V by an auxiliary voltage supply line, in turn supplied by a voltage line L through a conventional pantograph P. Alternatively, it may be possible to arrange for such components to be powered by an electrical supply from a battery or from a fuel cell, in a manner known per se and thus not shown or described in detail.

The air quality sensor 18 is adapted to measure a concentration of particulate matter, an ozone concentration, and a concentration of carbon dioxide in the air at said outlet opening 24, to generate a respective measurement signal Sp representative of said measurements, and to transmit it by means of data transmission (known per se, not shown) to the electronic control unit ECU. These data transmission means may alternatively be wired or wireless.

Preferably, the air quality sensor 18 comprises a multichannel optical sensor, which allows the continuous and simultaneous determination of the total particulate matter, the PM10 particulate fraction, the PM4 particulate fraction, the 2.5 particulate fraction, and the PM1 particulate fraction, or at least two of the above, in addition to measuring the concentration of carbon dioxide and of ozone.

Advantageously, the purification and microbiological sanitization system 10 further comprises at least one environmental sensor 28 adapted to measure at least one of either the temperature or humidity of the air within said purification and microbiological sanitization chamber 20, to generate a respective measurement signal Se representative of such measurement and to transmit it to said electronic control unit ECU. In a manner known per se, it is possible to use a plurality of these environmental sensors 28, both to measure the different environmental parameters at different points of the purification and microbiological sanitization chamber 20 and to measure each environmental parameter separately, or, alternatively but in an equivalent manner, it is possible to use a single integrated environmental sensor 28 adapted to measure a plurality of environmental parameters. Lastly, the environmental sensor 28 may also be adapted to detect further parameters, such as the air pressure, or the concentration of other components, such as for example volatile organic components, or any other polluting components.

Advantageously, the purification and microbiological sanitization system 10 further comprises at least one anemometer 30 adapted to measure the air velocity within said purification and microbiological sanitization chamber 20, preferably at or near said outlet opening 24 of the casing 14, to generate a respective measurement signal Sv representative of said measurement, and to transmit it to said electronic control unit ECU.

Clearly, it is also possible that the air quality sensor 18, the environmental sensor 28, and the anemometer 30 are made (all or at least two thereof) integrally in a single measurement component.

In a further embodiment, inside the purification and microbiological sanitization chamber 20 there is also a wire mesh filtering device 32, which is heated by means of a dedicated heater, preferably electrically powered, and even more preferably with recovery of the waste heat energy of the light-emitting diodes LED, and which is adapted to heat the air contained or passing through the purification and microbiological sanitization chamber 20, preferably up to a maximum temperature of about 55-65°C, only near the grid of the wire mesh. Preferably, this wire mesh filtering device is provided in the form of a resistance wire mesh and is arranged in such a way as to be in contact with metal heat sinks associated with the light-emitting diodes LED to recover the heat thereby dissipated.

As mentioned above, the purification and microbiological sanitization system 10 further comprises the electronic control unit ECU. Said electronic control unit ECU is configured, or programmed, to control the purification and microbiological sanitization system 10, or at least one of the components that comprise it.

In particular, according to the invention, the electronic control unit ECU is configured to control the radiant power of at least one light-emitting diode LED as a function of the measurement signal Sp received from the air quality sensor 18.

In a preferable embodiment of the invention, the electronic control unit ECU carries out the aforesaid control of the purification and microbiological sanitization system 10, or of at least one of the components that comprise it, by controlling the frequency of the supply current (PWM signal).

Advantageously, the electronic control unit ECU is configured to control the radiant power of at least one light-emitting diode LED as a function of the result of a comparison between the measurement signal Sp transmitted by the air quality sensor 18 and a control value stored in the electronic control unit ECU. The control value may thus be a programmed target value, whereby, for example, the radiant power of at least one light-emitting diode LED is increased if the target value has not been reached, and therefore the measurement signal Sp sent by the air quality sensor 18 is indicative that a concentration of particulate matter or a concentration of carbon dioxide is still higher than the target value. Conversely, if, following the outcome of this comparison, the electronic control unit ECU determines that the measurement signal Sp sent by the air quality sensor 18 is indicative of a concentration of particulate matter or a concentration of carbon dioxide sufficiently below the target value, then, preferably, the electronic control unit ECU may be configured to command a reduction in the radiant power emitted by at least one light-emitting diode LED in order to always ensure the minimum use of electrical power, and therefore improve the energy efficiency of the purification and microbiological sanitization system 10 as a whole.

Advantageously, the electronic control unit ECU may also be configured to control an electrical power supply to the ionizer device 16 as a function of the measurement signal Sp transmitted by the air quality sensor 18.

Advantageously, the electronic control unit ECU may also be configured to control the radiant power of at least one light-emitting diode LED and/or the electric power supply to the ionizer device 16 also as a function of the measurement signal Se transmitted from the environmental sensor 28, when present.

Lastly, advantageously, the electronic control unit ECU may also be configured to control the radiant power of at least one light-emitting diode LED and/or to control the electrical power supply to the ionizer device 16 also as a function of the measurement signal Sv transmitted by said anemometer 30, when present.

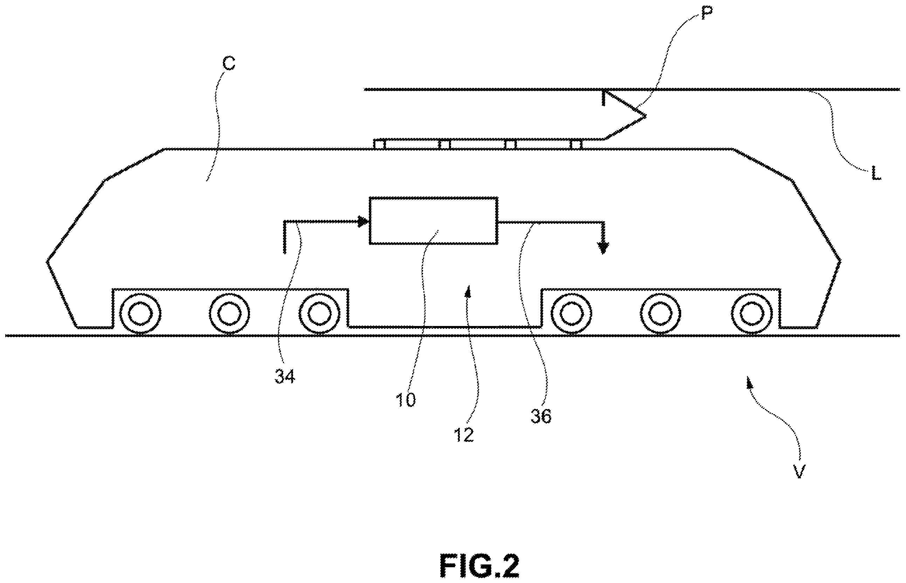

As mentioned above, the purification and microbiological sanitization system 10 is adapted to be applied to the heating, ventilation, or air conditioning system 12 of a railway vehicle V, or of a single coach C of said railway vehicle V, as shown in Fig. 2. The system 12 comprises, in addition to the purification and microbiological sanitization system 10 according to the invention, an inflow duct 34 and an outflow duct 36. The inflow duct 34 and the outflow duct 36 are made as conventional ducts of a ventilation, heating, or air conditioning system and therefore are not described in further detail.

The inflow duct 34, in any case, is adapted to take in air to be sanitized and purified, or decontaminated, from the coach C of the railway vehicle V and conduct it to said purification and microbiological sanitization system 10, to introduce it into the purification and microbiological sanitization chamber 20 of the purification and microbiological sanitization system 10 through the inlet opening 22. Similarly, and in the opposite manner, the outflow duct 36 is adapted to reintroduce the sanitized and purified, or decontaminated, air coming from the purification and microbiological sanitization system 10 into the coach C of the railway vehicle V.

In a known manner, the heating, ventilation, or air conditioning system 10 may comprise additional ventilation, heating, or air conditioning elements, which may be arranged both upstream and downstream of the purification and microbiological sanitization system 10.

Naturally, without prejudice to the principle of the invention, the embodiments and the details of construction may vary widely with respect to that which has been described and illustrated purely by way of non-limiting example, without thereby departing from the scope of the invention defined by the appended claims.

In particular, the arrangement or position of the component elements of the purification and microbiological sanitization system 10 may be modified without thereby departing from the scope of the invention as defined in the appended claims. It is possible, for example, to arrange, alternatively, the ionizer device 16 downstream or upstream of the filter 26, and, similarly, to arrange the light-emitting diodes LED downstream from, coinciding with, or upstream of the ionizer device 16, it being understood that that which is shown in the figures and described in this description constitutes one or some of the embodiments of the invention falling under the scope of the appended claims.

## Claims

1. Air purification and microbiological sanitization system (10) for a heating, ventilation, or air conditioning system (12) of a railway vehicle (V), or the like, the purification and microbiological sanitization system (10) comprising:
a casing (14), adapted to define therein a purification and microbiological sanitization chamber (20), and having an inlet opening (22) adapted to allow the inflow of air into the purification and microbiological sanitization chamber (20) and an outlet opening (24) adapted to allow the outflow of air from the purification and microbiological sanitization chamber (20);
an ionizer device (16) adapted to ionize at least partially the air contained within said purification and microbiological sanitization chamber (20) of the casing (14);
an electronic control unit (ECU) configured to control the purification and microbiological sanitization system (10); and
an air quality sensor (18) adapted to measure a particulate matter concentration and a carbon dioxide concentration in the air at said outlet opening (24), to generate a respective measurement signal (Sp) representative of said measurements, and to transmit it to said electronic control unit (ECU),
**characterized in that** it further comprises
a plurality of light-emitting diodes (LED) each adapted to generate a germicidal ultraviolet radiation having a wavelength between 100 nanometers and 300 nanometers and to emit said radiation inside said purification and microbiological sanitization chamber (20) of the casing (14);
wherein the electronic control unit (ECU) is configured to control the radiant power of at least one light-emitting diode (LED) as a function of the measurement signal (Sp) transmitted by the air quality sensor (18),
wherein said purification and microbiological sanitation chamber (20) is divided into a plurality of ducts (21) arranged side by side, and
wherein at least one respective light-emitting diode (LED) of said plurality of light-emitting diodes (LED) is arranged inside each of said ducts (21).

2. Purification and microbiological sanitizing system according to claim 1, wherein the light-emitting diodes (LEDs) of said plurality of light-emitting diodes (LEDs) are connected in series with each other.

3. Purification and microbiological sanitization system according to claim 1 or claim 2, wherein the at least one respective light-emitting diode (LED) arranged inside each of said ducts (21) is arranged at a mid-section of the respective duct (21).

4. Purification and microbiological sanitization system according to any of the preceding claims, wherein the electronic control unit (ECU) is configured to control the radiant power of at least one light-emitting diode (LED) as a function of the outcome of a comparison between the measurement signal (Sp) transmitted by the air quality sensor (18) and a control value stored in the electronic control unit (ECU).

5. Purification and microbiological sanitization system according to claim or to claim 4, wherein the air quality sensor (18) is adapted to measure simultaneously and continuously the carbon dioxide concentration, the ozone concentration and the concentration of at least two among: the total particulate, the PM10 particulate fraction, the PM4 particulate fraction, the PM2.5 particulate fraction, and the PM1 particulate fraction.

6. Purification and microbiological sanitization system according to any of the preceding claims, wherein the inner surface of each duct (21) of said plurality of ducts (21) of the purification and microbiological sanitization chamber (20) is coated with an ultraviolet radiation reflective material UV-C, preferably aluminum and/or polytetrafluoroethylene.

7. Purification and microbiological sanitization system according to any of the preceding claims, wherein the electronic control unit (ECU) is further configured to control an electrical power supply to the ionizer device (16) as a function of the respective measurement signal (Sp) transmitted by the air quality sensor (18).

8. Purification and microbiological sanitization system according to any of the preceding claims, further comprising an environmental sensor (28) adapted to measure at least one of either the temperature or the humidity of the air inside said purification and microbiological sanitization chamber (20), to generate a respective measurement signal (Se) representative of said measurement, and to transmit it to said electronic control unit (ECU), wherein the electronic control unit (ECU) is further configured to control the radiant power of said at least one light-emitting diode (LED) and/or to control an electrical power supply to the ionizer device (16) also as a function of the measurement signal (Se) transmitted by the environmental sensor (28).

9. Purification and microbiological sanitization system according to any of the preceding claims, further comprising an anemometer (30) adapted to measure the air speed inside said purification and microbiological sanitization chamber (20), to generate a respective measurement signal (Sv) representative of said measurement, and to transmit it to said electronic control unit (ECU), wherein the electronic control unit (ECU) is further configured to control the radiant power of said at least one light-emitting diode (LED) and/or to control an electrical power supply to the ionizer device (16) also as a function of the measurement signal (Sv) transmitted by said anemometer (30).

10. Purification and microbiological sanitization system according to any of the preceding claims, further comprising a filtering device (32) with a wire mesh, arranged inside said purification and microbiological sanitization chamber (20) and adapted to heat the air contained therein.

11. Purification and microbiological sanitization system according to any of the preceding claims, further comprising a class G4 filter (26), according to the European EN 779:201 standard, preferably arranged at the inlet opening (22) of the casing (14) and adapted to filter air flowing into the purification and microbiological sanitization chamber (20).

12. Purification and microbiological sanitization system according to claim 11, wherein the ionizer device (16), the filter (26), and the plurality of ducts (21) are arranged in such a way that, during operation, the air introduced into the microbiological sanitization and purification chamber (20) passes through the ionizer device (16), the filter (26), and then the plurality of ducts (21) in sequence.

13. Heating, ventilation, or air conditioning system (12) of a railway vehicle (V) comprising:
a purification and microbiological sanitization system (10) according to any of the preceding claims;
an air inflow duct (34) adapted to draw air to be sanitized and purified from a coach (C) of the railway vehicle (V) and to conduct it to said purification and microbiological sanitization system (10) in order to introduce it into the purification and microbiological sanitization chamber (20) of the purification and microbiological sanitization system (10) by means of said inlet opening (22); and
an air outflow duct (36) adapted to re-introduce sanitized and purified air coming from the purification and microbiological sanitization system (10) inside said coach (C) of the railway vehicle (V).

## Patentansprüche

1. Luftreinigungs- und mikrobiologisches Desinfektionssystem (10) für ein Heizungs-, Belüftungs- oder Klimatisierungssystem (12) eines Schienenfahrzeugs (V) oder dergleichen, wobei das Reinigungs- und mikrobiologische Desinfektionssystem (10) aufweist:
ein Gehäuse (14), das dazu ausgelegt ist, eine Reinigungs- und mikrobiologische Desinfektionskammer (20) darin zu definieren, und das eine Einlassöffnung (22) aufweist, die dazu ausgestaltet ist, das Einströmen von Luft in die Reinigungs- und mikrobiologische Desinfektionskammer (20) zu ermöglichen, sowie eine Auslassöffnung (24), die dazu ausgestaltet ist, das Ausströmen von Luft aus der Reinigungs- und mikrobiologischen Desinfektionskammer (20) zu ermöglichen;
eine Ionisierungsvorrichtung (16), die dazu ausgestaltet ist, die innerhalb der Reinigungs- und mikrobiologischen Desinfektionskammer (20) des Gehäuses (14) enthaltene Luft zumindest teilweise zu ionisieren;
eine elektronische Steuereinheit (ECU), die dazu ausgelegt ist, das Luftreinigungs- und mikrobiologische Desinfektionssystem (10) zu steuern; und
einen Luftqualitätssensor (18), der dazu ausgestaltet ist, eine Partikelkonzentration und eine Kohlendioxidkonzentration in der Luft bei der Auslassöffnung (24) zu messen, ein entsprechendes Messsignal (Sp), das für die Messungen repräsentativ ist, zu erzeugen und es an die elektronische Steuereinheit (ECU) zu übermitteln,
**dadurch gekennzeichnet, dass** es weiterhin
eine Mehrzahl von lichtemittierenden Dioden (LED) aufweist, die jeweils dazu ausgestaltet sind, eine keimtötende ultraviolette Strahlung mit einer Wellenlänge zwischen 100 und 300 nm zu erzeugen und diese Strahlung ins Innere der Reinigungs- und mikrobiologischen Desinfektionskammer (20) des Gehäuses (14) zu emittieren;
wobei die elektronische Steuereinheit (ECU) dazu ausgelegt ist, die Strahlungsleistung zumindest einer lichtemittierenden Diode (LED) in Abhängigkeit von dem von dem Luftqualitätssensor (18) übermittelten Messsignal (Sp) zu steuern,
wobei die Reinigungs- und mikrobiologische Desinfektionskammer (20) in eine Mehrzahl von Seite an Seite angeordneten Kanälen (21) unterteilt ist, und
wobei mindestens eine der jeweiligen lichtemittierenden Dioden (LED) der Mehrzahl von lichtemittierenden Dioden (LED) innerhalb jedes der Kanäle (21) angeordnet ist.

2. Reinigungs- und mikrobiologisches Desinfektionssystem nach Anspruch 1, wobei die lichtemittierenden Dioden (LEDs) der Mehrzahl von lichtemittierenden Dioden (LEDs) miteinander in Reihe geschaltet sind.

3. Reinigungs- und mikrobiologisches Desinfektionssystem nach Anspruch 1 oder Anspruch 2, wobei die mindestens eine der jeweiligen innerhalb jedes der Kanäle (21) angeordneten lichtemittierenden Dioden (LED) in einem Mittelbereich des jeweiligen Kanals (21) angeordnet ist.

4. Reinigungs- und mikrobiologisches Desinfektionssystem nach einem der vorangehenden Ansprüche, wobei die elektronische Steuereinheit (ECU) dazu ausgelegt ist, die Strahlungsleistung mindestens einer lichtemittierenden Diode (LED) in Abhängigkeit von dem Ergebnis eines Vergleichs zwischen dem von dem Luftqualitätssensor (18) übermittelten Messsignal (Sp) und einem in der elektronischen Steuereinheit (ECU) gespeicherten Kontrollwert zu steuern.

5. Reinigungs- und mikrobiologisches Desinfektionssystem nach Anspruch [*Angabe fehlt; Anmerkung des Übersetzers*] oder Anspruch 4, wobei der Luftqualitätssensor (18) dazu ausgestaltet ist, gleichzeitig oder kontinuierlich die Kohlendioxidkonzentration, die Ozonkonzentration und die Konzentration zumindest zweier Partikel aus den Gesamtpartikeln, der Partikelfraktion PM10, der Partikelfraktion PM4, der Partikelfraktion PM2,5 und der Partikelfraktion PM1 zu messen.

6. Reinigungs- und mikrobiologisches Desinfektionssystem nach einem der vorangehenden Ansprüche, wobei die Innenfläche jedes Kanals (21) der Mehrzahl von Kanälen (21) der Reinigungs- und mikrobiologischen Desinfektionskammer (20) mit einem ultraviolette Strahlung reflektierenden Material UV-C, bevorzugt Aluminium und/oder Polytetrafluorethylen, beschichtet ist.

7. Reinigungs- und mikrobiologisches Desinfektionssystem nach einem der vorangehenden Ansprüche, wobei die elektronische Steuereinheit (ECU) weiter dazu ausgelegt ist, eine elektrische Energieversorgung an die Ionisierungsvorrichtung (16) in Abhängigkeit von dem von dem Luftqualitätssensor (18) übermittelten Messsignal (Sp) zu steuern.

8. Reinigungs- und mikrobiologisches Desinfektionssystem nach einem der vorangehenden Ansprüche, weiter aufweisend einen Umgebungssensor (28), der dazu ausgestaltet ist, mindestens eines von entweder der Temperatur oder der Feuchtigkeit der Luft innerhalb der Reinigungs- und mikrobiologischen Desinfektionskammer (20) zu messen, ein entsprechendes Messsignal (Sp), das für die Messungen repräsentativ ist, zu erzeugen und es an die elektronische Steuereinheit (ECU) zu übermitteln, wobei die elektronische Steuereinheit (ECU) weiter dazu ausgelegt ist, die Strahlungsleistung der mindestens einen lichtemittierenden Diode (LED) zu steuern und/oder eine elektrische Energieversorgung an die Ionisierungsvorrichtung (16) auch in Abhängigkeit von dem von dem Umgebungssensor (28) übermittelten Messsignal (Sp) zu steuern.

9. Reinigungs- und mikrobiologisches Desinfektionssystem nach einem der vorangehenden Ansprüche, weiter aufweisend ein Anemometer (30), das dazu ausgestaltet ist, die Luftgeschwindigkeit innerhalb der Reinigungs- und mikrobiologischen Desinfektionskammer (20) zu messen, ein entsprechendes Messsignal (Sp), das für die Messungen repräsentativ ist, zu erzeugen und es an die elektronische Steuereinheit (ECU) zu übermitteln, wobei die elektronische Steuereinheit (ECU) weiter dazu ausgelegt ist, die Strahlungsleistung der mindestens einen lichtemittierenden Diode (LED) zu steuern und/oder eine elektrische Energieversorgung an die Ionisierungsvorrichtung (16) auch in Abhängigkeit von dem von dem Anemometer (30) übermittelten Messsignal (Sp) zu steuern.

10. Reinigungs- und mikrobiologisches Desinfektionssystem nach einem der vorangehenden Ansprüche, weiter aufweisend eine Filtervorrichtung (32) mit einem Drahtgeflecht, die innerhalb der Reinigungs- und mikrobiologischen Desinfektionskammer (20) angeordnet ist und dazu ausgestaltet ist, die darin enthaltene Luft zu erwärmen.

11. Reinigungs- und mikrobiologisches Desinfektionssystem nach einem der vorangehenden Ansprüche, weiter aufweisend einen Filter der Klasse G4 (26) gemäß dem europäischen Standard EN 779:201, der vorzugsweise an der Einlassöffnung (22) des Gehäuses (14) angeordnet und dazu ausgestaltet ist, in die Reinigungs- und mikrobiologischen Desinfektionskammer (20) einströmende Luft zu filtern.

12. Reinigungs- und mikrobiologisches Desinfektionssystem nach Anspruch 11, wobei die Ionisierungsvorrichtung (16), der Filter (26) und die Mehrzahl von Kanälen (21) derart angeordnet sind, dass im Betrieb die in die Reinigungs- und mikrobiologische Desinfektionskammer (20) eingeführte Luft nacheinander die Ionisierungsvorrichtung (16), den Filter (26) und die Mehrzahl von Kanälen (21) passiert.

13. Heizungs-, Belüftungs- oder Klimatisierungssystem (12) eines Schienenfahrzeugs (V), aufweisend:
ein Reinigungs- und mikrobiologisches Desinfektionssystem (10) nach einem der vorangehenden Ansprüche;
einen Luftzuführkanal (34), der dazu ausgestaltet ist, zu desinfizierende und zu reinigende Luft von einem Wagen (C) des Schienenfahrzeugs (V) einzuziehen und sie dem Reinigungs- und mikrobiologisches Desinfektionssystem (10) zuzuleiten, um sie mittels der Einlassöffnung (22) in die Reinigungs- und mikrobiologische Desinfektionskammer (20) des Reinigungs- und mikrobiologischen Desinfektionssystem (10) einzuleiten; und
einen Luftableitkanal (36), der dazu ausgestaltet ist, desinfizierte und gereinigte aus dem Reinigungs- und mikrobiologischen Desinfektionssystem (10) kommende Luft wieder in das Innere des Wagens (C) des Schienenfahrzeugs (V) einzuleiten.

## Revendications

1. Système de purification et de désinfection microbiologique (10) d'air pour un système de chauffage, ventilation ou climatisation (12) d'un véhicule ferroviaire (V) ou autre, le système de purification et de désinfection microbiologique (10) comprenant :
un boîtier (14), adapté pour définir dans celui-ci une chambre de purification et de désinfection microbiologique (20), et ayant une ouverture d'entrée (22) adaptée pour permettre l'admission d'air dans la chambre de purification et de désinfection microbiologique (20) et une ouverture de sortie (24) adaptée pour permettre l'évacuation d'air de la chambre de purification et de désinfection microbiologique (20) ;
un dispositif ioniseur (16) adapté pour ioniser au moins partiellement l'air contenu dans ladite chambre de purification et de désinfection microbiologique (20) du boîtier (14) ;
une unité de commande électronique (ECU) configurée pour commander le système de purification et de désinfection microbiologique (10) ; et
un capteur de qualité d'air (18) adapté pour mesurer une concentration en matières particulaires et une concentration en dioxyde de carbone dans l'air au niveau de ladite ouverture de sortie (24), pour générer un signal de mesure (Sp) respectif représentatif desdites mesures, et pour le transmettre à ladite unité de commande électronique (ECU),
**caractérisé en ce qu'**il comprend en outre
une pluralité de diodes électroluminescentes (LED) chacune adaptée pour générer un rayonnement ultraviolet germicide ayant une longueur d'onde entre 100 nanomètres et 300 nanomètres et pour émettre ledit rayonnement à l'intérieur de ladite chambre de purification et de désinfection microbiologique (20) du boîtier (14) ;
dans lequel l'unité de commande électronique (ECU) est configurée pour commander la puissance rayonnante d'au moins une diode électroluminescente (LED) en fonction du signal de mesure (Sp) transmis par le capteur de qualité d'air (18),
dans lequel ladite chambre de purification et de désinfection microbiologique (20) est divisée en une pluralité de conduits (21) agencés côte à côte, et
dans lequel au moins une diode électroluminescente (LED) respective de ladite pluralité de diodes électroluminescentes (LED) est agencée à l'intérieur de chacun desdits conduits (21).

2. Système de purification et de désinfection microbiologique selon la revendication 1, dans lequel les diodes électroluminescentes (LED) de ladite pluralité de diodes électroluminescentes (LED) sont connectées en série les unes aux autres.

3. Système de purification et de désinfection microbiologique selon la revendication 1 ou 2, dans lequel l'au moins une diode électroluminescente (LED) respective agencée à l'intérieur de chacun desdits conduits (21) est agencée au niveau d'une section médiane du conduit (21) respectif.

4. Système de purification et de désinfection microbiologique selon l'une quelconque des revendications précédentes, dans lequel l'unité de commande électronique (ECU) est configurée pour commander la puissance rayonnante d'au moins une diode électroluminescente (LED) en fonction du résultat d'une comparaison entre le signal de mesure (Sp) transmis par le capteur de qualité d'air (18) et une valeur de commande stockée dans l'unité de commande électronique (ECU).

5. Système de purification et de désinfection microbiologique selon l'une quelconque des revendications précédentes, dans lequel le capteur de qualité d'air (18) est adapté pour mesurer simultanément et de manière continue la concentration en dioxyde de carbone, la concentration en ozone et la concentration d'au moins deux parmi : le total des particules, la fraction de particules PM10, la fraction de particules PM4, la fraction de particules PM2,5 et la fraction de particules PM1.

6. Système de purification et de désinfection microbiologique selon l'une quelconque des revendications précédentes, dans lequel la surface intérieure de chaque conduit (21) de ladite pluralité de conduits (21) de la chambre de purification et de désinfection microbiologique (20) est revêtue d'un matériau réfléchissant le rayonnement ultraviolet UV-C, de préférence de l'aluminium et/ou du polytétrafluoroéthylène.

7. Système de purification et de désinfection microbiologique selon l'une quelconque des revendications précédentes, dans lequel l'unité de commande électronique (ECU) est en outre configurée pour commander une alimentation en courant électrique vers le dispositif ioniseur (16) en fonction du signal de mesure (Sp) respectif transmis par le capteur de qualité d'air (18).

8. Système de purification et de désinfection microbiologique selon l'une quelconque des revendications précédentes, comprenant en outre un capteur environnemental (28) adapté pour mesurer au moins une parmi soit la température soit l'humidité de l'air à l'intérieur de ladite chambre de purification et de désinfection microbiologique (20), pour générer un signal de mesure (Se) respectif représentatif de ladite mesure, et pour le transmettre à ladite unité de commande électronique (ECU), dans lequel l'unité de commande électronique (ECU) est en outre configurée pour commander la puissance rayonnante de ladite au moins une diode électroluminescente (LED) et/ou pour commander une alimentation en courant électrique vers le dispositif ioniseur (16) également en fonction du signal de mesure (Se) transmis par le capteur environnemental (28).

9. Système de purification et de désinfection microbiologique selon l'une quelconque des revendications précédentes, comprenant en outre un anémomètre (30) adapté pour mesurer la vitesse de l'air à l'intérieur de ladite chambre de purification et de désinfection microbiologique (20), pour générer un signal de mesure (Sv) respectif représentatif de ladite mesure, et pour le transmettre à ladite unité de commande électronique (ECU), dans lequel l'unité de commande électronique (ECU) est en outre configurée pour commander la puissance rayonnante de ladite au moins une diode électroluminescente (LED) et/ou pour commander une alimentation en courant électrique vers le dispositif ioniseur (16) également en fonction du signal de mesure (Sv) transmis par ledit anémomètre (30).

10. Système de purification et de désinfection microbiologique selon l'une quelconque des revendications précédentes, comprenant en outre un dispositif de filtrage (32) avec un tamis, agencé à l'intérieur de ladite chambre de purification et de désinfection microbiologique (20) et adapté pour chauffer de l'air contenu dans celle-ci.

11. Système de purification et de désinfection microbiologique selon l'une quelconque des revendications précédentes, comprenant en outre un filtre (26) de classe G4, selon la norme européenne EN 779:201, de préférence agencé au niveau de l'ouverture d'entrée (22) du boîtier (14) et adapté pour filtrer de l'air s'écoulant dans la chambre de purification et de désinfection microbiologique (20).

12. Système de purification et de désinfection microbiologique selon la revendication 11, dans lequel le dispositif ioniseur (16), le filtre (26) et la pluralité de conduits (21) sont agencés de telle manière que, durant le fonctionnement, l'air introduit dans la chambre de purification et de désinfection microbiologique (20) passe à travers le dispositif ioniseur (16), le filtre (26), et ensuite la pluralité de conduits (21) les uns après les autres.

13. Système de chauffage, ventilation ou climatisation (12) d'un véhicule ferroviaire (V) comprenant :
un système de purification et de désinfection microbiologique (10) selon l'une quelconque des revendications précédentes ;
un conduit d'admission d'air (34) adapté pour aspirer de l'air devant être désinfecté et purifié à partir d'un wagon (C) du véhicule ferroviaire (V) et pour le conduire vers ledit système de purification et de désinfection microbiologique (10) afin de l'introduire dans la chambre de purification et de désinfection microbiologique (20) du système de purification et de désinfection microbiologique (10) au moyen de ladite ouverture d'entrée (22) ; et
un conduit d'évacuation d'air (36) adapté pour réintroduire de l'air désinfecté et purifié provenant du système de purification et de désinfection microbiologique (10) à l'intérieur dudit wagon (C) du véhicule ferroviaire (V).
